# NOUVEAU FASCICULE DE BREVET EUROPEEN

(11) **EP 1 707 189 B2**
(45) Date de publication et mention de la décision concernant l'opposition: **26.07.2017**
(45) Mention de la délivrance du brevet: 12.03.2014
(21) Numéro de dépôt: 06013216.4
(22) Date de dépôt: 20.09.2000
(51) Int. Cl.: A61K 8/97, A61K 8/37, A61K 8/49, A61K 8/63, A61K 8/67, A61K 8/68, A61K 8/92, A23L 33/105, A23L 33/115, A61Q 19/10, A61Q 19/00, A61K 36/26, A61K 36/28, A61K 36/48, A61K 36/54, A61K 45/06

(54) **Utilisation d'un produit d'huile de tournesol pour traiter la peau**
Verwendung von einem Produkt aus pflanzlichem Öl zur Erhöhung der hautlipiden Produktion
Use of a vegetable oil product as agent for increasing skin lipid synthesis

(30) Priorité: 22.09.1999 FR 9911844
(43) Date de publication de la demande: 04.10.2006
(62) Demande divisionnaire de: 00964325.5
(73) Titulaire: Laboratoires Expanscience, 92400 Courbevoie (FR)
(72) Inventeur: Msika, Philippe, 78000 Versailles (FR); Piccirilli, Antoine, 78000 Versailles (FR)
(74) Mandataire: Regimbeau

(56) Documents cités:
- DE-A- 19 652 522
- FR-A- 2 471 775
- FR-A- 2 692 783
- FR-A2- 2 488 507
- US-A- 4 454 159

## Description

La présente invention se rapporte à l'utilisation d'un oléodistillat d'huile de tournesol en tant qu'agent pour augmenter la synthèse des lipides cutanés, notamment des lipides de la barrière cutanée épidermique, dans ou pour la préparation d'une composition cosmétique, pharmaceutique ou dermatologique. L'invention se rapporte également à une méthode de traitement cosmétique pour augmenter la synthèse des lipides cutanés, notamment des lipides de la barrière cutanée épidermique, et à l'utilisation du produit végétal comme additif alimentaire.

La peau est principalement constituée de trois couches : l'épiderme, le derme et l'hypoderme.

La couche la plus externe, l'épiderme, est caractérisée par une organisation en strates correspondant à un état de différenciation croissant des kératinocytes, de la zone la plus profonde (stratum basale) à la zone la plus superficielle (stratum corneum) au sein de laquelle des éléments anuclées (cornéocytes) sont inclus dans une structure lipidique extracellulaire multilamellaire, le ciment intercornéocytaire, responsable de la fonction de barrière hydrique de la peau et de protection contre les agressions extérieures.

Les corps lamellaires ou de Oddland, secrétés par le stratum granulosum, couche intermédiaire entre le stratum basale et le stratum corneum, contiennent du cholestérol, des phospholipides et des glucosylcéramides ainsi que des hydrolysases sélectives. Ces enzymes convertissent les phospholipides et les glucosylcéramides en acides gras libres et céramides qui forment, avec le cholestérol et le sulfate de cholestérol, les bicouches lamellaires intercellulaires du stratum corneum. Les céramides participent de façon prépondérante à la formation de la barrière constitué par le stratum corneum et à la régulation des flux hydriques en solidarisant les lamelles. Une diminution importante du contenu en/et du type de céramide est notamment observée dans la dermatite atopique (ou eczéma atopique) ou dans l'acné (céramide 1) et dans les peaux sèches et prurits des personnes âgées. Le sulfate de cholestérol, via une sulfatase spécifique, est en équilibre avec le cholestérol (agent de fluidité des feuillets). Ils jouent un rôle important dans la cohésion des cornéocytes et donc dans la desquamation cutanée, ainsi que dans le confort cutané.

L'altération de cette barrière cutanée provoquée par des agressions extérieures (rayonnement U.V, vent, froid, détergents etc.), par le phénomène naturel et inexorable du vieillissement et/ou par des dysfonctionnements pathologiques ou non (peaux sensibles, irritées, ou réactives) se traduit par une perturbation de l'homéostasie épidermique qu'il est souhaitable de pouvoir prévenir et/ou traiter tant sur le plan cosmétique que pharmaceutique et notamment dermatologique.

Par exemple, l'article de Ruby Ghadially et al, "Decreased Epidermal Lipid Synthesis Accounts for Altered Barrier Function in Aged Mice", The Journal Of Investigate Dermatology, Vol. 106, N°5, May 1996, enseigne qu'une barrière cutanée altérée ainsi qu'une teneur anormale en lipides dans un épiderme agé de souris peut s'expliquer par une synthèse altérée des lipides épidermiques.

Il existe donc un besoin de pouvoir stimuler la synthèse des lipides cutanés, notamment des lipides de la barrière cutanée épidermique, afin de pouvoir en particulier restaurer la fonction de barrière cutanée de l'épiderme et/ou de lutter contre les divers troubles cutanés liées à une baisse de la synthèse des lipides cutanés, notamment des lipides de la barrière cutanée épidermique.

Le brevet FR 2 692 783 décrit des compositions à base d'insaponifiables d'huile de germe de blé en association avec des insaponifiables d'huile de sésame. Le mélange d'insaponifiables d'huile de sésame et d'insaponifiables d'huile de germe de blé présente une activité curative contre les effets des UVA, mais le texte spécifie bien que la vitamine E seule ou chacun des insaponifiables mentionnés ci-dessus ne présente pas d'action curative contre ces effets.

Le brevet DE 196 52 522 présente un procédé de concentration de tocophérols et / ou stérols à partir de mélanges de graisses et / ou des dérivés de matières grasses, dans lequel le mélange est soumis à une distillation fractionnée et une distillation moléculaire. Ce document cite l'utilisation de produits contenant de la vitamine E pour leur activité anti-oxydante.

Le brevet US 4,454,159 décrit des compositions pour le traitement dermatologique des peaux irritées, sèches. Lesdites compositions sont des mélanges de nombreux produits tels que des combinaisons de lipides/lipoïdes ayant des propriétés antioxydantes et/ou riches en acides gras spécifiques.

Le brevet FR 2 471 775 présente une nouvelle huile utilisable en cosmétologie contenant un mélange d'huile de jojoba et d'huile de tournesol ainsi qu'une fraction insaponifiable, notamment de soja ou d'avocat, et/ou de l'huile de pistache.

On a maintenant trouvé de manière tout à fait surprenante et inattendue que l'utilisation de certains produits d'huile de tournesol permet d'augmenter avantageusement la synthèse des lipides cutanés, notamment des lipides de la barrière cutanée épidermique.

La présente invention a ainsi pour objet l'utilisation d'au moins un oléodistillat d'huile de tournesol selon les revendications 1 ou 5.

En particulier, l'utilisation selon l'invention est caractérisée en ce que les lipides cutanés sont choisis entre autres parmi les lipides épidermiques du groupe constitué par le cholestérol, le sulfate de cholestérol, les céramides 1 et 2 et les mélanges de ces derniers.

Par "oléodistillat d'huile de tournesol", on entend selon l'invention une huile de tournesol ayant été soumise à une étape de concentration de sa fraction insaponifiable.

L'insaponifiable est la fraction d'un corps gras qui, après action prolongée d'une base alcaline, reste insoluble dans l'eau et peut être extraite par un solvant organique. Cinq grands groupes de substances sont présents dans la plupart des insaponifiables d'huiles végétales : hydrocarbures saturés ou insaturés, alcools aliphatiques ou terpéniques, stérols, phytostérols, tocophérols, les pigments caroténoïdes et xanthophiles.

Les huiles de tournesol dont l'oléodistillat est riche en tocophérols et/ou en phytostérols sont particulièrement préférées pour l'utilisation selon l'invention. L'homme du métier comprend aisément que le terme "riche" fait référence à des teneurs en tocophérols et en phytostérols respectivement au-dessus des teneurs moyennes respectives obtenues en considérant les huiles de tournesol connues de l'homme du métier.

Différentes méthodes peuvent être utilisées pour concentrer la fraction insaponifiable d'une huile végétale : cristallisation par le froid, extraction liquide-liquide, distillation moléculaire.

La distillation moléculaire est particulièrement préférée, étant réalisée de préférence à une température comprise entre environ 180 et environ 260 °C en maintenant une pression comprise entre environ 10⁻³ et environ 10⁻² mmHg et de préférence de l'ordre de 10⁻³ mmHg. La concentration en insaponifiable du distillat peut atteindre 60%.

Cette distillation moléculaire, ainsi que toute autre distillation moléculaire pour la préparation des produits d'huile de tournesol à utiliser selon l'invention, comme décrit ci-après, est de préférence réalisée en utilisant un dispositif choisi parmi les distillateurs moléculaires de type centrifuge et les dispositifs moléculaires de type à film raclé.

Les distillateurs moléculaires de type centrifuge sont connus de l'homme du métier. Par exemple, la demande EP- 0 493 144 décrit un distillateur moléculaire de ce type. D'une manière générale, le produit à distiller est étalée en couche mince sur la surface chauffée (surface chaude) d'un rotor conique tournant à grande vitesse. L'enceinte de distillation est placée sous vide. Dans ces conditions, il y a évaporation et non pas ébullition, depuis la surface chaude, des constituants de l'insaponifiable, l'avantage étant que l'huile et l'insaponifiable (ces produits étant réputés fragiles) ne sont pas dégradés au cours de l'évaporation.

Les distillateurs moléculaires de type à film raclé, également connus de l'homme du métier, comprennent une chambre de distillation dotée d'un racleur tournant, permettant l'étalement en continu sur la surface d'évaporation (surface chaude) du produit à distiller. Les vapeurs de produit sont condensées par le biais d'un doigt réfrigéré, placé au centre de la chambre de distillation. Les systèmes périphériques d'alimentation et de vide sont très proches de ceux d'un distillateur centrifuge (pompes d'alimentation, pompes à vide à palette et à diffusion d'huile,etc.). La récupération des résidus et des distillats dans des ballons en verre, se fait par écoulement gravitationnel.

Selon la présente invention, on utilise un oléodistillat d'huile de tournesol.

De préférence, l'oléodistillat de tournesol est obtenu par distillation moléculaire d'une huile de tournesol alimentaire. Les conditions de distillation sont de préférence les suivantes :
- température de 230 à 250 °C;
- pression de 10⁻³ à 10⁻² mmHg;
- taux de distillation d'environ 5 à 10 % massique.

Le taux de distillation peut être défini de la manière suivante : il s'agit du rapport massique ramené à 100 % de la masse du distillat à la somme (masse du distillat + masse du résidu).

Le distillat ainsi obtenu, c'est à dire l'oléodistillat de tournesol, présente une teneur en insaponifiable comprise entre environ 6 et environ 10 % en poids, la partie restante étant composée par les triglycérides de l'huile de tournesol.

Plusieurs procédés ont été décrits dans l'art antérieur pour extraire la fraction insaponifiable d'une huile végétale.

On peut citer en particulier le procédé de préparation d'insaponifiable d'huile d'avocat tel que décrit et revendiqué dans le brevet FR-2 678 632 au nom des Laboratoires Pharmascience. Ce procédé permet d'obtenir un insaponifiable d'avocat riche en fraction H en comparaison aux procédés classiques de préparation d'insaponifiable d'avocat.

On peut également citer le procédé de préparation d'insaponifiable d'huile de soja, obtenu à partir d'un concentrat d'insaponifiable d'huile de soja. Ledit concentrat d'insaponifiable est préparé par distillation moléculaire selon un procédé tel que décrit pour l'huile de lupin dans la demande de brevet FR-2 762 512, mais adapté à l'huile de soja. Dans ce procédé, l'huile de soja est distillée dans un distillateur moléculaire de type centrifuge ou à film raclé, à une température comprise entre environ 210 et 250° C et sous un vide poussé, compris entre 0.01 et 0.001 millimètres de mercure (soit 0,13 à 1,3 Pa). Le distillat obtenu présente une teneur en insaponifiable comprise entre 5 et 30% en poids et constitue donc un concentrat d'insaponifiable d'huile de soja. Le même concentrat est ensuite saponifié selon un procédé classique de saponification, en présence de potasse éthanolique. Le mélange obtenu est extrait par le dichloroéthane dans une colonne à contre-courant. La phase solvant est enfin désolvantée par passage dans un évaporateur à film tombant afin de récupérer l'insaponifiable de soja.

L'oléodistillat d'huile de tournesol tel que décrit ci-dessus est utilisé selon une proportion comprise entre environ 0,01 et 100 % en poids, de préférence entre environ 0,5 et environ 10 % en poids, par rapport au poids total de la composition.

Le milieu cosmétiquement, pharmaceutiquement ou dermatologiquement acceptable peut être tout milieu adapté pour les formes galéniques connues de l'homme du métier, en vue d'une administration par voie topique ou orale.

En particulier, ce milieu peut être une solution huileuse, une émulsion eau-dans-huile, une émulsion huile-dans eau, une microémulsion, un gel huileux, un gel anhydre, une dispersion de vésicules, de microcapsules ou de microparticules.

De préférence, la composition pour l'utilisation selon l'invention est adaptée pour une administration par application topique.

L'effet avantageux d'augmentation de la synthèse des lipides cutanés, notamment des lipides de la barrière cutanée épidermique, permet de prévenir et/ou de traiter, en d'autres termes permet le traitement des altérations de la barrière cutanée formée principalement par les couches épidermiques du stratum coméum et granulosum comme expliqué ci-dessus.

Ainsi, l'utilisation selon l'invention est caractérisée en ce que la composition est destinée au traitement des peaux sèches et des peaux ayant été soumises à un rayonnement actinique, notamment un rayonnement U.V. tel que le rayonnement solaire ou le rayonnement d'une lampe U.V par exemple lors d'une séance de bronzage artificiel.

L'utilisation selon l'invention est également caractérisée en ce que la composition est destinée au traitement de l'ichtyose, de l'acné, de la xérose, de la dermatite atopique (ou eczéma atopique), des troubles de la desquamation cutanée, des peaux sensibles, irritées et réactives et du prurit.

La présente invention a encore pour objet une utilisation cosmétique selon la revendication 5 des troubles liés au vieillissement de la peau, des muqueuses voisines et/ou des phanères, caractérisée en ce qu'on applique sur la peau, les muqueuses voisines et/ou les phanères une composition contenant au moins un oléodistillat d'huile de tournesol dans un milieu cosmétiquement acceptable tels que décrits ci-dessus.

L'invention a par ailleurs pour objet une utilisation cosmétique selon la revendication 5 des troubles liés au dessèchement de la peau, des muqueuses voisines et/ou des phanères, caractérisée en ce qu'on applique sur la peau, les muqueuses voisines et/ou les phanères une composition contenant au moins un oléodistillat d'huile de tournesol dans un milieu cosmétiquement acceptable tels que décrits ci-dessus.

Selon un mode de mise en oeuvre préféré l'oléodistillat d'huile de tournesol est présent dans la composition selon une proportion comprise entre environ 0,01 et 100 % en poids, de préférence entre environ 0,5 et environ 10 % en poids, par rapport au poids total de la composition.

Enfin, l'invention a encore pour objet l'utilisation d'au moins un oléodistillat d'huile de tournesol, tel que décrit ci-dessus, en tant qu'additif dans un aliment pour l'être humain et/ou l'animal.

Cette utilisation alimentaire est de préférence caractérisée en ce que l'oléodistillat d'huile de tournesol est présent dans l'aliment selon une proportion comprise entre environ 0,1 et environ 20 % en poids, par rapport au poids total de l'aliment.

Les exemples suivants sont destinés à illustrer la présente invention et ne doivent en aucun cas être interprétés comme pouvant en limiter la portée.

A moins qu'il n'en soit précisé autrement, les pourcentages indiqués dans les exemples suivants sont des pourcentages en poids.

### Exemple 1 : préparation de produits d'huile végétale et leur utilisation selon l'invention sous forme d'émulsions huiles-dans-eau

Les compositions 1.1 à 1.3 et la composition placébo suivantes ont chacune été préparé de la manière suivante :
Les composants constituant la phase aqueuse (eau et glycérine) sont placées au bain-marie à 75 °C. Les composants de la phase grasse, à l'exception du SEPIGEL 305, du SILICONE SF 1202 et du produit d'huile végétale (respectivement préparés ci-après sous les dénominations "Oléodistillat de tournesol-1", "Insaponifiables-1" et "Lipides-1"), sont placés au bain-marie à 75 °C. Juste avant de réaliser l'émulsification, on ajoute à la phase grasse le SILICONE 1202 et le produit d'huile végétale respectif. On réalise ensuite l'émulsion sous turbine à vitesse lente par incorporation de la phase grasse dans la phase aqueuse. Lorsque la préparation atteint la température de 60 °C, on ajoute le SEPIGEL 305 sous turbine à grande vitesse. On laisse ensuite refroidir la composition au repos jusqu'à température ambiante, avant son utilisation dans les essais décrits ci-après.

### 1.1) Composition 1.1 : utilisation d'un oléodistillat d'huile de tournesol

On prépare un oléodistillat de tournesol par distillation moléculaire dans un distillateur moléculaire de type centrifuge d'une huile de tournesol alimentaire du commerce. Les conditions de distillation sont les suivantes :
- température 220 °C;
- pression de 10⁻³ mmHg;
- taux de distillation : 6,7 % massique
- débit d'alimentation : 18 kg/h

Le distillat obtenu, l'oléodistillat de tournesol, présente une teneur en insaponifiable d'environ 6,2 % en poids, la partie restante étant composée par les triglycérides de l'huile de tournesol. L'Oléodistillat ainsi obtenu est dénommé "Oléodistillat de tournesol-1 ".

| **Composition 1.1 (formule INCI)** | **% (en poids)** |
|---|---|
| **Phase aqueuse** | |
| Eau | 67,3 |
| Glycérine | 4 |
| **Phase grasse** | |
| Sorbitan Tristearate | 1,85 |
| Peg 40 Stearate | 3,15 |
| Silicone SF 1202 | 3 |
| Cetiol Oe | 1 |
| Vaseline Codex | 2,5 |
| Glycéryl Stearate | 6 |
| Decyl Pentanoate | 3 |
| **Oléodistillat de tournesol-1** | 2 |
| Beeswax | 3 |
| Stearate Peg 2 | 1 |
| C12-15 Alcool Benzoate | 1 |
| Phenonip | 0,7 |
| Sepigel 305 | 0,5 |
| **TOTAL** | **100%** |

### 1.2) Composition comparative 1.2 : utilisation d'un mélange d'un insaponifiable d'avocat et de soja

On utilise le mélange d'insaponifiables d'huiles d'avocat et de soja tel que commercialisé par la société Laboratoires Pharmascience sous la dénomination "Piascledine 300^{®}" qui consiste en un mélange de 33,3% en poids d'insaponifiable d'avocat et de 66,6% en poids d'insaponifiable de soja, par rapport au poids total du mélange (les 0,1 % restants étant constitués de silice colloïdale et de butylhydroxytoluène). Ce mélange est dénommé ci-après "Insaponifiables-1".

| **Composition 1.2 (noms INCI)** | |
|---|---|
| **Phase aqueuse** | |
| Eau | 67,3 |
| Glycérine | 4 |
| **Phase grasse** | |
| Sorbitan Tristearate | 1,85 |
| Peg 40 Stearate | 3,15 |
| Silicone SF 1202 | 3 |
| Cetiol Oe | 1 |
| Vaseline Codex | 2,5 |
| Glycéryl Stearate | 6 |
| Decyl Pentanoate | 3 |
| **Insaponifiables-1** | 2 |
| Beeswax | 3 |
| Stearate Peg 2 | 1 |
| C12-15 Alcool Benzoate | 1 |
| Phenonip | 0,7 |
| Sepigel 305 | 0,5 |
| **TOTAL** | **100 %** |

### 1.3) Composition comparative 1.3 : utilisation de lipides furaniques d'avocat

On prépare un insaponifiable d'avocat comme décrit dans le brevet FR-2 678 632. Sa composition est la suivante :
- alcools gras polyhydroxylés 24,3 %
- lipides furaniques 55,5 %
- stérols 3,1 %
- squalène 1,4 %
- autres 15,7 % (1)
(1) acides gras libres, hydrocarbures, tocophérols, cétones grasses et pigments lourds

On soumet cet insaponifiable à une distillation moléculaire à l'aide du distillateur moléculaire à film raclé commercialisé par la société Leybold sous la dénomination "KDL4". Les conditions de distillation sont les suivantes :
- température surface chaude: 108°C
- pression : 10⁻³ mm Hg
- vitesse de rotation de l'arbre: 240 t/min.
- débit d'insaponifiable d'avocat: 400 ml/h

### Rendement en distillat: 48,6 %

### Composition du distillat:

- alcools gras Polyhydroxylés : n.m.
- lipides furaniques 99,1 %
- stérols n.m.
- squalène n.m.
- autres 0,9 % (1)
(1) acides gras libres, hydrocarbures et cétones grasses
("n.m." : non mesurable, c'est à dire une teneur inférieure à 0,05 %)

Il s'agit donc d'un distillat très riche en lipides furaniques dans la mesure où la teneur de ces derniers excède 99 %. Ce distillat est dénommé par la suite "Lipides-1 ".

| **Composition 1.3 (noms INCI)** | **% (en poids)** |
|---|---|
| **Phase aqueuse** | |
| Eau | 69 |
| Glycérine | 4 |
| **Phase grasse** | |
| Sorbitan Tristearate | 1,85 |
| Peg 40 Stearate | 3,15 |
| Silicone SF 1202 | 3 |
| Cetiol Oe | 1 |
| Vaseline Codex | 2,5 |
| Glycéryl Stearate | 6 |
| Decyl Pentanoate | 3 |
| **Lipides-1** | 0,3 |
| Beeswax | 3 |
| Stearate Peg 2 | 1 |
| C 12-15 Alcool Benzoate | 1 |
| Phenonip | 0,7 |
| Sepigel 305 | 0,5 |
| **TOTAL** | **100%** |

### 1.4) Composition placébo

| **Composition placébo (noms INCI)** | **% (en poids)** |
|---|---|
| **Phase aqueuse** | |
| Eau | 69,3 |
| Glycérine | 4 |
| **Phase grasse** | |
| Sorbitan Tristearate | 1,85 |
| Peg 40 Stearate | 3,15 |
| Silicone SF 1202 | 3 |
| Cetiol Oe | 1 |
| Vaseline Codex | 2,5 |
| Glycéryl Stearate | 6 |
| Decyl Pentanoate | 3 |
| Beeswax | 3 |
| Stearate Peg 2 | 1 |
| C12-15 Alcool Benzoate | 1 |
| Phenonip | 0,7 |
| Sepigel 305 | 0,5 |
| **TOTAL** | **100%** |

### Exemple 2 : évaluation in vitro de l'effet des compositions 1.1, 1.2, 1.3, et placébo sur le métabolisme des lipides épidermiques dans un modèle organotypique de peau humaine entière en culture

Dans ce qui suit, on utilise les abréviations suivantes:
- EGF :: facteur de croissance épidermique, *Epidermal growth factor;*
- CCM :: chromatographie en couche mince;
- MCF: : milieu de culture des disques de peau humaine;
- MIF: : milieu d'incubation des disques de peau humaine;
- PBS: : tampon phosphate salin, *phosphate buffered saline.*

L'objet de cette étude est d'étudier l'effet des quatre compositions 1.1, 1.2, 1.3 et placébo décrites ci-dessus sur le métabolisme des lipides épidermiques.

L'étude est réalisée *in vitro* dans un modèle organotypique de peau humaine entière en culture. Deux techniques mises en oeuvre successivement :
- mesure de l'incorporation d'acétate radiomarqué au carbone 14 dans le *totum* des lipides épidermiques néosynthétisés ;
- analyse en chromatographie en couche mince pour séparer les principales classes de lipides épidermiques radiomarqués néosynthétisés.

L'effet des produits à l'essai est comparé à celui observé en présence du facteur de croissance épidermique (EGF), dilué dans le milieu de culture des disques de peau humaine, et en présence d'une formulation cosmétique commercialisée contenant de l'acide lactique. L'EGF et l'acide lactique stimulent tous deux de manière connue la synthèse des céramides par les kératinocytes (Ponec M. Gibbs S., Weerheim A., Kempenaar J., Mulder A. and Mommaas A.M. - Epidermal growth factor and temperature regulate keratinocytes differentiation - Arch. Dermatol. Res., 1997, 289, 317-326; et Rawlings A.V., Davies A., Carlomusto M., Pillai S. Ahang K., Kosturbo R., Verdejo P., Feinberg C., Nguyen L. and Chandar P. - Effect of lactic acid isomers on keratinocyte ceramide synthesis, stratum corneum lipid levels and stratum corneum barrier function - Arch. Dermatol. Res., 1996, 288, 383-390).

### 1) Matériels et méthode

### 1.1) Produits à l'essai, produits de référence et réactifs

Les compositions 1.1, 1.2, 1.3, et placébo ont été préparées comme décrit ci-dessus. L'EGF provenait de chez R&D SYSTEMS. La formulation cosmétique contenant de l'acide lactique, appelée par la suite "acide lactique", a été achetée dans le réseau de distribution grandes surfaces.

La solution de rinçage des disques de peau humaine après l'incubation est le tampon PBS : NaCl 8g/l ; Na₂HPO₄ 1,15g/l ; KH₂PO₄ 0,2g/l ; KCl 0,2g/l ; CaCl₂ 0,1g/l ; MgCl₂ 0,1g/l ; pH 7,4.

Les autres réactifs, de qualité analytique, proviennent de chez CARLO ERBA, GIBCO et SIGMA, sauf indication contraire.

### 1.2) Système d'essai

Un fragment de peau humaine a été collecté après une opération de plastie abdominale. Celle-ci a été réalisée chez une femme âgée de 24 ans (sujet I0129). Des disques de peau de 8 mm de diamètre ont été découpés à l'aide d'un emporte-pièce.

Les disques de peau sont déposés dans des nacelles. Les nacelles sont placées dans des puits de culture contenant le milieu MCF, composé du milieu MEM/M199 (3/4, 1/4 ; v/v) additionné de pénicilline (50 UI/ml), de streptomycine (50 µg/ml), de bicarbonate de sodium (0,2 %, p/v) et de SVF (2 %, v/v).

### 1.3) Incubation des produits à l'essai et des produits de référence avec le système d'essai

Les produits à l'essai sont testés non dilués. Ils sont déposés au centre de chaque disque de peau humaine, à raison de 10 mg/cm². Le milieu d'incubation des disques de peau humaine (milieu MIF) est composé du milieu MCF contenant 1 µCi/ml d'acétate marqué au carbone 14 (AMERSHAM, activité spécifique : 57 mCi/mmole).

L'EGF est testé à 10 ng/ml dans le milieu MIF. L'acide lactique est utilisée en application topique (10 mg/cm²).

Les disques de peau humaine sont incubés en présence des produits à l'essai et des produits de référence pendant 18 heures à 37°C dans une atmosphère humide contenant 5% de CO₂.

Des disques de peau témoins sont incubés en parallèle en absence de produits à l'essai et de produits de référence.

Chaque condition expérimentale est réalisée en *quadriplicate.*

L'échelle de temps suivante est utilisée : 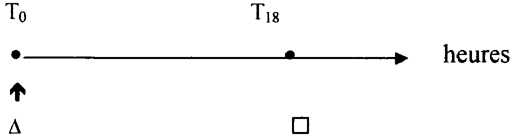
↑: application topique des produits à l'essai et du produit de référence et dilution de l'EGF dans le milieu d'incubation des disques de peau
Δ : ajout d'acétate marqué au carbone 14 dans le milieu de culture
□ : dissociation derme/épiderme et évaluation des effets

### 1.4) Evaluation des effets

### 1.4.1) Néosynthèse des lipides épidermiques totaux

A la fin de l'incubation, les disques de peau humaine sont abondamment rincés avec le tampon PBS. L'épiderme de chaque disque de peau est dissocié du derme par un choc thermique contrôlé (eau MilliQ, 2 min, 62°C). Les épidermes ainsi dissociés sont digérés par la trypsine (ICN, 1%, p/v) pendant une nuit à 37°C. La dissociation cellulaire est facilitée par action des ultrasons.

Les lipides néosynthétisés, marqués au carbone 14, sont extraits par partition entre une phase organique (méthanol/chloroforme, 1/4, v/v) et une phase aqueuse (chlorure de potassium à 0,25 M). La phase organique est évaporée sous azote et les résidus sont repris dans un mélange chloroforme/méthanol, 2/1 (v/v).

La radioactivité de chaque échantillon, correspondant à la quantité d'acétate incorporé dans les lipides néosynthétisés, est mesurée en scintillation liquide.

Les résultats sont exprimés en cpm/mg d'épiderme.

### 1.4.2) Nature des lipides épidermiques néosynthétisés

A partir des échantillons de lipides extraits, des aliquotes de 20µl, correspondant à 3700 cpm, sont déposées sur des plaques de chromatographie de silice 60 (MERCK). Celles-ci sont développées dans trois solvants successifs :
- chloroforme/acétone/méthanol, 38/2/10 (v/v/v),
- chloroforme/acétone/méthanol, 40/5/5 (v/v/v),
- chloroforme/acétate d'éthyl/éther/méthanol, 36/10/3/1 (v/v/v/v).

Ce système permet de séparer le sulfate de cholestérol, les cérébrosides, les céramides, le cholestérol et les tri- + di-glycérides. Les lipides plus polaires, appelés par la suite "lipides polaires", restent au point de dépôt.

Les plaques de silice sont ensuite mises en exposition avec des films pour autoradiographie pendant 15 jours (AMERSHAM, Hyperfilm beta max).

La position des différentes classes de lipides- lipides polaires, sulfate de cholestérol, cérébrosides, céramides 1 et 2, cholestérol et tri- + di-glycérides - est déterminée à l'aide des standards appropriés.

La radioactivité des spots séparés et révélés grâce à l'autoradiographie est comptée avec un analyseur de radioactivité argon-méthane sur couche mince (BERTHOLD). Les résultats sont exprimés en pourcentages de la radioactivité des lipides totaux néosynthétisés et déposés sur la CCM.

### 1.5) Traitement des données

Les groupes de données (groupe témoin et groupes traités) sont comparés par une analyse de la variance à un facteur (ANOVA 1, p<0,05), suivie par un test de Dunnett.

### 2) Résultats

Après une nuit d'incubation en présence des disques de peau humaine, les compositions à l'essai et les compositions de référence n'ont pas d'effet significatif sur la néosynthèse des lipides épidermiques totaux (tableau 3.1). En revanche, ils modifient de façon notable la proportion des différents lipides épidermiques dans ce *totum* :
- l'**EGF** à 10 ng/ml diminuait de 46% la néosynthèse du sulfate de cholestérol et augmentait de 55% la néosynthèse du céramide 2 (tableau 3.2);
- l'**acide lactique** augmente d'un facteur 1,59 la néosynthèse des cérébrosides (tableau 3.2);
- la **composition 1.1** augmente d'un facteur 2,26 et 4,61 la néosynthèse respective des céramides 1 et 2, d'un facteur 5,04 la néosynthèse du cholestérol. Il diminue de 73% la néosynthèse des tri- et di-glycérides (tableau 3.3);
- la **composition 1.2** augmente d'un facteur 1,32 la néosynthèse du sulfate de cholestérol, d'un facteur 2,47 et 2,51 la néosynthèse respective des céramides 1 et 2, d'un facteur 4,62 la néosynthèse du cholestérol. Il diminue de 77% la néosynthèse des tri- et di-glycérides (tableau 3.2);
- la **composition 1.3** augmente d'un facteur 1,24 la néosynthèse du sulfate de cholestérol, d'un facteur 1,59 et 3,66 la néosynthèse respective des céramides 1 et 2, d'un facteur 4,14 la néosynthèse du cholestérol. Il diminue de 84% la néosynthèse des tri- et di-glycérides (tableau 3.2);
- la **composition placébo** diminue de 59% la néosynthèse des tri- et di-glycérides (tableau 3.3.) sans provoquer d'augmentation significative des différents lipides épidermiques analysés.

En conclusion, dans les conditions expérimentales retenues, les composition 1.1, 1.2, 1.3, et placébo n'ont pas d'effet significatif sur la néosynthèse des lipides épidermiques totaux.

Ils modifient par contre de façon significative la proportion des différentes classes des lipides épidermiques séparés en chromatographie en couche mince dans le *totum.*

Ils diminuent la néosynthèse des tri- et di-glycérides au profit des autres lipides épidermiques.

La composition 1.1 augmente la néosynthèse du cholestérol (sans modification de la néosynthèse du sulfate de cholestérol) et la néosynthèse des céramides.

Les compositions 1.2 et 1.3 augmentent la néosynthèse du sulfate de cholestérol et du cholestérol, et la néosynthèse des céramides.

La composition placébo ne provoque pas d'augmentation significative des différents lipides épidermiques analysés.

### 3) Tableaux de résultats

**Tableau 3.1) : Effet des compositions 1.1, 1.2, 1.3, et placébo ainsi que de l'EGF et d'une formulation cosmétique contenant de l'acide lactique sur la néosynthèse des lipides épidermiques totaux dans des disques de peau humaine entière, après 18 heures d'incubation**

| Témoin | EGF 10 ng/ml | Acide lactique | Composition 1.2 | Composition 1.3 | Composition 1.1 | Composition placébo |
|---|---|---|---|---|---|---|
| 4183,35 | 3104,63 | 1864,31 | 2479,26 | 3646,42 | 873,10 | 1506,12 |
| 4407,89 | 5043,66 | 4919,12 | 3858,70 | 5255,70 | 3726,00 | 4154,88 |
| 3691,69 | 3606,76 | 4027,67 | 6571,13 | 3900,53 | 3802,63 | 4429,96 |
| 1062,72 | 4565,02 | 1076,21 | 2457,78 | 3209,16 | 905,55 | 2373,94 |
| **3336,41** | **4080,02** | **2971,83** | **3841,72** | **4002,95** | **2326,82** | **3116,23** |
| +/- | +/- | +/- | +/- | +/- | +/- | +/- |
| **1545,02** | **883,02** | **1800,62** | **1934,04** | **882,63** | **1660,22** | **1408,09** |
| *100* | *122* | *89* | *115* | *120* | *70* | *93* |

| | | | | | | |
|---|---|---|---|---|---|---|
| Les résultats sont exprimés en cpm/mg d'épiderme. En gras : moyenne et écart type En italique : pourcentage du groupe témoin * : moyenne significativement différente du groupe témoin (p<0,05) | | | | | | |

**Tableau 3.2) : Effet des compositions 1.2 et 1.3 ainsi que de l'EGF et d'une formulation cosmétique contenant de l'acide lactique sur la néosynthèse des lipides polaires, du sulfate de cholestérol, des cérébrosides, des céramides 1 et 2, du cholestérol et des tri- + di-glycérides dans des disques de peau humaine entière, après 18 heures d'incubation**

| Produit | Lipides polaires | Sulfate de cholestérol | Cérébrosides | Céramide 1 | Céramide 2 | Cholestérol | Tri- + di-glycérides |
|---|---|---|---|---|---|---|---|
| | 28,94 | 6,04 | 1,89 | 2,03 | 2,03 | 8,23 | 50,83 |
| | 24,96 | 4,80 | 2,08 | 2,67 | 2,07 | 5,04 | 53,76 |
| | 31,74 | 6,26 | 4,02 | 2,71 | 2,15 | 7,89 | 45,23 |
| Témoin | 42,90 | 5,12 | 4,44 | 1,67 | 1,47 | 5,61 | 38,84 |
| | **32,14** | **5,56** | **3,11** | **2**,**27** | **1,93** | **6,69** | **47,17** |
| | +/- | +/- | +/- | +/- | +/- | +/- | +/- |
| | **7,70** | **0,71** | **1,31** | **0,51** | **0,31** | **1,60** | **6,58** |
| | 100 | 100 | 100 | 100 | 100 | 100 | 100 |
| | 57,27 | 2,31 | 2,87 | 2,02 | 3,81 | 7,07 | 42,67 |
| | 34,42 | 3,69 | 3,82 | 1,41 | 2,38 | 9,46 | 44,82 |
| | 27,69 | 3,44 | 4,77 | 3,41 | 2,74 | 7,86 | 50,08 |
| EGF 10 ng/ml | 33,53 | 2,57 | 4,02 | 4,02 | 3,05 | 9,99 | 42,83 |
| | **38,23** | **3,00*** | **3,87** | **2,72** | **3,00*** | **8,60** | **45,10** |
| | +/- | +/- | +/- | +/- | +/- | +/- | +/- |
| | **13,04** | **0,67** | **0,78** | **1,21** | **0,61** | **1,36** | **3,46** |
| | 119 | 54 | 125 | 120 | 155 | 128 | 96 |
| | 49,20 | 4,07 | 4,46 | 2,37 | 1,70 | 7,77 | 33,43 |
| | 33,04 | 4,16 | 4,54 | 2,64 | 3,32 | 9,72 | 42,58 |
| | 64,49 | 5,90 | 6,07 | 2,94 | 2,57 | 8,07 | 31,95 |
| Acide Lactique | 53,51 | 4,25 | 4,68 | 2,58 | 1,44 | 8,67 | 24,86 |
| | **50,06** | **4,60** | **4,94*** | **2,63** | **2,26** | **8,56** | **33,21** |
| | +/- | +/- | +/- | +/- | +/- | +/- | +/- |
| | **13,05** | **0,87** | **0,76** | **0,24** | **0,86** | **0,86** | **7,28** |
| | 156 | 83 | 159 | 116 | 117 | 128 | 70 |
| | 24,57 | 6,89 | 4,41 | 2,78 | 6,86 | 44,35 | 10,13 |
| | 28,71 | 8,34 | 5,91 | 5,94 | 3,34 | 35,04 | 12,72 |
| Composition 1.2 | 33,09 | 6,86 | 4,02 | 4,55 | 4,79 | 36,97 | 9,72 |
| | 35,78 | 7,35 | 7,76 | 9,20 | 4,37 | 34,01 | 11,52 |
| | **30,54** | **7,36*** | **5,53** | **5,62*** | **4,84*** | **37,59*** | **11,02*** |
| | +/- | +/- | +/- | +/- | +/- | +/- | +/- |
| | **4,93** | **0,69** | **1,70** | **2,72** | **1,48** | **4,67** | **1,37** |
| | 95 | 132 | 178 | 247 | 251 | 562 | 23 |
| | 38,88 | 6,23 | 6,23 | 3,20 | 8,92 | 28,63 | 7,92 |
| | 43,70 | 8,25 | 4,55 | 3,51 | 7,25 | 24,34 | 8,41 |
| Composition 1.3 | 37,70 | 6,88 | 3,97 | 3,52 | 6,69 | 33,55 | 7,68 |
| | 47,94 | 6,15 | 6,13 | 4,24 | 5,40 | 24,30 | 5,85 |
| | **42,06** | **6,88*** | **5,22** | **3,62*** | **7,07*** | **27,71** | **7,47*** |
| | +/- | +/- | +/- | +/- | +/- | +/- | +/- |
| | **4,70** | **0,97** | **1,13** | **0,44** | **1,46** | **4,39** | **1,12** |
| | 131 | 124 | 168 | 159 | 366 | 414 | 16 |

| | | | | | | | |
|---|---|---|---|---|---|---|---|
| Les résultats sont exprimés en pourcentage des lipides totaux radiomarqués déposés sur la CCM. En gras : moyenne et écart type En italique : pourcentage du groupe témoin * : moyenne significativement différente du groupe témoin (p<0,05) | | | | | | | |

**Tableau 3.3) : Effet des compositions 1.1 et palcébo sur la néosynthèse des lipides polaires, du sulfate de cholestérol, des cérébrosides, des céramides 1 et 2, du cholestérol et des tri- + di-glycérides dans des disques de peau humaine entière, après 18 heures d'incubation**

| Produit | Lipides polaires | Sulfate de cholestérol | Cérébrosides | Céramide 1 1 | Céramide 2 | Cholestérol | Tri- + di-glycérides |
|---|---|---|---|---|---|---|---|
| | 28,94 | 6,04 | 1,89 | 2,03 | 2,03 | 8,23 | 50,83 |
| | 24,96 | 4,80 | 2,08 | 2,67 | 2,07 | 5,04 | 53,76 |
| | 31,74 | 6,26 | 4,02 | 2,71 | 2,15 | 7,89 | 45,23 |
| Témoin | 42,90 | 5,12 | 4,44 | 1,67 | 1,47 | 5,61 | 38,84 |
| | **32,14** | **5,56** | **3,11** | **2,27** | **1,93** | **6,69** | **47,17** |
| | +/- | +/- | +/- | +/- | +/- | +/- | +/- |
| | **7,70** | **0,71** | **1,31** | **0,51** | **0,31** | **1,60** | **6,58** |
| | 100 | 100 | 100 | 100 | 100 | 100 | 100 |
| | 37,24 | 6,43 | 4,35 | 5,12 | 8,38 | 32,19 | 16,28 |
| | 32,48 | 4,83 | 5,45 | 4,22 | 7,77 | 32,51 | 12,74 |
| Composition 1.1 | 31,92 | 3,81 | 5,68 | 5,43 | 8,06 | 32,23 | 10,86 |
| | 25,87 | 4,41 | 4,49 | 5,75 | 11,37 | 38,00 | 10,11 |
| | **31,88** | **4,87** | **4,99** | **5,13^{*}** | **8,90*** | **33,73*** | **12,50*** |
| | +/- | +/- | +/- | +/- | +/- | +/- | +/- |
| | **4,66** | **1,12** | **0,67** | **0,66** | **1,67** | **2,85** | **2,75** |
| | 99 | 88 | 161 | 226 | 461 | 504 | 26 |
| | 73,67 | 9,27 | 7,69 | 2,44 | 2,05 | 3,03 | 18,60 |
| | 58,70 | 11,03 | 7,60 | 2,51 | 3,39 | 3,71 | 13,07 |
| Composition placébo | 55,90 | 6,63 | 3,71 | 4,21 | 3,67 | 8,66 | 17,20 |
| | 63,39 | 5,69 | 4,48 | 4,17 | 3,35 | 4,93 | 27,98 |
| | **62,92** | **8,16** | **5,87** | **3,33** | **3,12** | **5,08** | **19**,**21*** |
| | +/- | +/- | +/- | +/- | +/- | +/- | +/- |
| | **7,81** | **2,44** | **2,07** | **0,99** | **0,72** | **2,51** | **6,30** |
| | 196 | 147 | 189 | 147 | 161 | 76 | 41 |

| | | | | | | | |
|---|---|---|---|---|---|---|---|
| Les résultats sont exprimés en pourcentage des lipides totaux radiomarqués déposés sur la CCM. En gras : moyenne et écart type En italique : pourcentage du groupe témoin * : moyenne significativement différente du groupe témoin (p<0,05) | | | | | | | |

### Exemple 3 : composition d'une crème pour peaux atopiques

| **Formule INCI** | % |
|---|---|
| Water | QSP 100 |
| Glycerin | 15 |
| Petrolatum | 2 |
| Hydrogenated Palm Kernel Oil | 5 |
| Caprilic/Capric Triglycérides | 5 |
| Cyclomethicone | 1 |
| Sucrose Distearate | 4 |
| Dextrin | 3 |
| Sunflower (Heliantus Annuus) seed Oil Unsaponifiables (1) | 1 |
| Squalane | 2 |
| Candelilla (Euphorbia Cerifera) Wax | 1 |
| Sucrose Stearate | 2 |
| Oat (Avena Sativa) Flour | 1 |
| Dimethiconol | 0,2 |
| Methylparaben | 0,4 |
| Propylparaben | 0,3 |
| Xanthan Gum | 0,2 |
| Ceramide 3 | 0,2 |
| **Total :** | **100%** |

| | |
|---|---|
| **(1)** : Oléodistillat de tournesol-1 de l'exemple 1 | |

### Exemple 4 : composition d'une huile pour le bain pour peaux atopiques

| **Formule INCI** | % |
|---|---|
| Sunflower (Helianthus Annuus) Seed Oil | QSP 100 |
| Octyl Cocoate | 15 |
| Sweet Almond (Prunus Amygdalus Dulcis) Oil | 15 |
| Mineral Oil | 1 |
| PEG-6 Isostearate | 5 |
| Sunflower (Helianthus Annuus) seed Oil Unsaponifiables (1) | 60 |
| Chamomile (Anthemis Nobilis) Oil | 5 |
| Propylene Glycol Dipelargonate | 1 |
| Lecithin | 1 |
| Laureth-2 | 0,5 |
| Tocopherol | 0,5 |
| Ascorbyl palmitate | 0,06 |
| **Total :** | **100 %** |

| | |
|---|---|
| **(1)** : Oléodistillat de tournesol-1 de l'exemple 1 | |

### Exemple 5 : étude clinique pour évaluation de l'effet de soin de la composition 1.1. de l'exemple 1 et vérification de sa bonne tolérance locale cutanée, sous controle dermatologigue, après applications uniques et répétées pendant 4 semaines, chez le volontaire adulte "atopique"

### 1) Matériel et Méthode

### 1.1 Objectif de l'Etude

Il s'agit d'évaluer, d'une part, l'effet de soin d'un produit cosmétique, par diverses mesures biométrologiques associées à des évaluations cliniques, et de vérifier d'autre part, leur bonne tolérance locale cutanée, après applications cutanées unique et répétées pendant 4 semaines, chez le volontaire adulte "atopique", à la peau du corps très sèche et squameuse.

### 1.2 Pertinence de l'essai

Evaluations, en "double aveugle", fondées sur :
- les principes de conductivité électrique de la peau, largement décrits pour déterminer l'état d'hydratation des couches supérieures de l'épiderme (Tagami H. et al., 1980 ; Korstanje et al., 1992);
- le principe de la photométrie (mesures sébumétriques), permettant d'évaluer l'effet relipidant d'un produit cosmétique;
- l'analyse en microscopie optique de "biopsies" de surface, réalisées par stripping, à l'aide de colle cyanoacrylate, permettant de déterminer l'effet d'un produit cosmétique sur le Réseau MicroDépressionnaire (R.M.D.);
- une évaluation clinique par le Directeur de l'Etude, une auto-évaluation par le panéliste et un questionnaire.

### 1.3 Critères d'inclusion

Volontaire "atopique" à la peau du corps sèche à très sèche et légèrement squameuse (score sécheresse ≥ 5, sur une échelle de 1 à 9).

### 1.4 Population étudiée

18 volontaires adultes de sexe féminin (ou 20 pour la cinétique), "atopiques", âgés de 20 à 28 ans, à la peau sèche ou très sèche (2 abandons non liés aux applications).

### 1.5 Modalités d'application.

Application unique : 0,07 ml de produit, soit 2 µl/cm², sur une ou deux zones de 35 cm² environ délimitée au niveau de la peau de la jambe droite ou gauche, selon une randomisation au hasard. Une zone témoin a également été délimitée pour chaque type de mesures (cornéométrie et sébumétrie).

Utilisations répétées : 2 fois par jour, dans les conditions normales d'utilisation, pendant 4 semaines consécutives, par le volontaire lui-même à son domicile, en hémi-corps.

### 1.6 Méthodologie

Application unique : mesure de la capacité électrique à l'aide d'un Cornéomètre™ (Courage + Khazaka electronic GmbH, Allemagne) et du taux initial de lipides cutanés de surface à l'aide du Sébumètre™ SM 810 PC (Courage et Khazaka) au niveau des zones traitées avec le produit étudié (cinétique d'hydratation seulement), ainsi qu'au niveau d'une zone témoin non traitée (une zone témoin par type de mesure) avant, puis 1, 2, 3 et 24 heures environ après l'application des produits.

Utilisations répétées :
- mesure de la capacité électrique à l'aide d'un Cornéomètre™ (Courage + Khazaka electronic GmbH, Allemagne) au niveau des zones traitées avec le produit étudié avant, puis après les 4 semaines d'utilisation du produit;
- réalisation de "biopsies" de surface par stripping, puis analyse par microscopie optique, selon des échelles linéaires semi-structurées de 12 cm, faisant état de la netteté du Réseau MicroDépressionnaire et de l'aspect de surface, avant puis après les 4 semaines d'applications.
- évaluation clinique par l'Investigateur de l'Etude et auto-évaluation par les panélistes, de la "sécheresse", de la "rugosité" et de la "desquamation" de la peau, sur la base de scores cliniques ou d'échelles visuelles analogiques, aux mêmes temps que précédemment ;
- appréciation de la tolérance locale cutanée du produit par le Dermatologue, après les 4 semaines d'utilisation ;
- température et humidité relative régulées et contrôlées à chaque temps de l'essai (T° = 22±2°C et HR = 50 ± 5%).

### 1.7 Statistiques

Mesures instrumentales (cornéométrie et sébumétrie) : ANOVA et test de comparaisons multiples (p < 0,05) portant sur les valeurs absolues et sur les différences (Δ Tx - T0).

R.M.D., Echelles analogiques et scores cliniques : test de Wilcoxon en séries appariées ("two-tail", p < 0,05).

Calcul des pourcentages de variation des paramètres évalués au cours de l'étude.

### 2) Résultats

### 2.1 EFFET SUR LE TAUX DE LIPIDES CUTANES DE SURFACE APRES APPLICATION UNIQUE (Sébumètre™)

On constate une élévation statistiquement significative du taux de lipides cutanés de surface par rapport aux mesures initiales et aux valeurs relevées au niveau de la zone témoin, 1 puis 2 et 3 heures environ après la première application, traduisant un net effet relipidant immédiat, non mis en évidence 24 heures environ après l'application (reflétant une absorption totale de la composition 1.1, sans présence de film gras résiduel à la surface de la peau).

### 2.2 EFFET SUR LE DEGRE D'HYDRATATION DES COUCHES SUPERIEURES DE L'EPIDERME APRES APPLICATIONS UNIQUE & REPETEES (Cornéomètre™)

### - Après application unique (n = 20)

On constate une élévation statistiquement significative de la capacité électrique par rapport aux mesures initiales et aux valeurs relevées au niveau de la zone témoin, 1 puis 2, 3 et 24 heures environ après la première application de la composition 1.1.

### - Après 4 semaines d'utilisations répétées (n = 18)

On constate une élévation statistiquement significative de la capacité électrique par rapport aux mesures initiales.

**TABLEAU 5.1**

| **GAINS D'HYDRATATION** | **Zone Témoin** | **COMPOSITION 1.1** **(EXEMPLE 1)** |
|---|---|---|
| **T 1 heure** | + 0,0% | **+ *48,6%* °** |
| **T 2 heures** | + 1,0% | **+ *58,7%* °** |
| **T 3 heures** | + 0,5% | **+ *64,4%* °** |
| **T 24 heures** | + 2,9% | + *34,2%* ° |
| **T 4 semaines** | | + *22,4%* |

| | | |
|---|---|---|
| ° *: augmentation statistiquement significative en comparaison à la zone témoin non traitée* | | |

### 2.3 EFFET SUR LE RESEAU MICRODEPRESSIONNAIRE

### (analyse en microscopie optique de "biopsie" de surface : échelles linéaires semi-structurées de 12 cm)

On constate une restructuration statistiquement significative du Réseau MicroDépressionnaire, après 4 semaines d'applications.

**TABLEAU 5.2**

| | **COMPOSITION 1.1** **(EXEMPLE 1)** |
|---|---|
| **Microrelief** | + *10%* |
| **Aspect de surface** | + *52%* |

### 2.4 EVALUATION CLINIQUE PAR LE DIRECTEUR DE L'ETUDE

### (scores cliniques en 9 points)

On observe une variation statistiquement significative des critères de jugement suivants, après 4 semaines d'application :

**TABLEAU 5.3**

| | **COMPOSITION 1.1** **(EXEMPLE 1)** |
|---|---|
| **Sécheresse de la pea**u | **-54%** *(p* = *0,0002)* |
| **Rugosité de la peau** | **-52%** *(p* = *0,0002)* |
| **Desquamation** | **-54%** *(p* = *0***,***0004)* |

### 2.5 AUTO-EVALUATION PAR LES VOLONTAIRES

### (échelles visuelles analogiques en 10 pointes)

On observe une variation statistiquement significative des critères de jugement suivants, après 4 semaines d'application :

**TABLEAU 5.4**

| | **COMPOSITION 1.1** **(EXEMPLE 1)** |
|---|---|
| **Sécheresse de la peau** | **-60%** *(p* = *0, 0002)* |
| **Rugosité de la peau** | **-55%** *(p* = *0,0002)* |
| **Desquamation** | **-60%** *(p* = *0,0002)* |

### 3) Conclusion

En conclusion, l'application cutanée unique de la composition 1.1 chez 20 volontaires adultes de sexe féminin "atopiques", à la peau sèche à très sèche, en comparaison à une zone témoin non traitée (en double aveugle), a entraîné :
- un effet statistiquement significatif sur le taux de lipides cutanés des surfaces (mesures photométriques), en comparaison à une zone témoin non traitée, traduisant un net effet relipidant immédiat, non mis en évidence 24 heures environ après l'application, reflétant une absorption totale du produit, sans présence de film gras résiduel à la surface de la peau;
- un effet marqué sur le degré d'hydratation des couches supérieures de l'épiderme (mesures de la capacité électrique), 1, 2, 3 et 24 heures environ après l'application, traduisant une excellente rémanence.

Les applications répétées, 2 fois par jour pendant 4 semaines consécutives, dans les conditions normales d'utilisation, par un panel de 18 sujets adultes de sexe féminin, ont, par ailleurs, entraîné :
- un effet statistiquement significatif sur le degré d'hydratation des couches supérieures de l'épiderme ;
- une restructuration statistiquement significative du réseau microdépréssionnaire ;
- une amélioration statistiquement significative de l'aspect de la peau (sécheresse, rugosité et desquamation).

Un jugement positif a également été formulé par la majorité des panélistes pour l'efficacité de la composition 1.1. en tant que "crème de soin pour peaux sèches", ainsi que pour ses qualités cosmétiques.

Les applications de la composition 1.1. étudiée se sont, par ailleurs, avérées très bien tolérées.

L'ensemble de ces résultats permet donc de justifier, pour la composition 1.1, les propriétés suivantes :
- un effet relipidant immédiat.
- un effet hydratant immédiat et longue durée des couches supérieures de l'épiderme,
- une amélioration de l'aspect de la peau ; et
- une tolérance et efficacité testées sous contrôle dermatologique.

## Revendications

1. Utilisation d'au moins un oléodistillat d'huile de tournesol pour la préparation d'une composition destinée au traitement des peaux sèches, des peaux ayant été soumises à un rayonnement actinique, notamment un rayonnement UV, des peaux sensibles, des peaux irritées, des peaux réactives, de la desquamation cutanée, du prurit, de l'ichtyose, de l'acné, de la xérose, ou de la dermatite atopique.

2. Utilisation selon la revendication 1, **caractérisée en ce que** les oléodistillats d'huile de tournesol sont choisis dans le groupe constitué par les oléodistillats riches en tocophérols et/ou en phytostérols.

3. Utilisation selon la revendication 1 ou 2, **caractérisée en ce que** la composition est destinée à une application topique.

4. Utilisation selon la revendication 1 ou 2, **caractérisée en ce que** la composition est destinée à une administration orale.

5. Utilisation cosmétique non thérapeutique d'au moins un oléodistillat d'huile de tournesol pour traiter les troubles liés au vieillissement de la peau, des muqueuses voisines et/ou des phanères ou pour traiter les troubles liés au dessèchement de la peau, des muqueuses voisines et/ou des phanères,.

6. Utilisation cosmétique selon la revendication 5, **caractérisée en ce que** l'oléodistillat d'huile de tournesol est destiné à une application topique.

7. Utilisation cosmétique selon la revendication 5, **caractérisée en ce que** l'oléodistillat d'huile de tournesol est destiné à une administration orale.

## Patentansprüche

1. Verwendung mindestens eines Öldestillats von Sonnenblumenöl für die Herstellung einer Zusammensetzung, die zur Behandlung von trockener Haut, Haut, die aktinischer Strahlung insbesondere UV-Strahlung, ausgesetzt worden ist, empfindlicher Haut, gereizter Haut, reaktiver Haut, Hautabschürfung, Pruritus, Ichthyose, Akne, Xerose oder atopischer Dermatitis.

2. Verwendung nach Anspruch 1, **dadurch gekennzeichnet, dass** die Öldestillate von Sonnenblumenöl ausgewählt sind aus der Gruppe bestehend aus Öldestillaten, die reich an Tocopherolen und/oder Phytosterolen sind.

3. Verwendung nach Anspruch 1 oder 2, **dadurch gekennzeichnet, dass** die Zusammensetzung für eine topische Anwendung bestimmt ist.

4. Verwendung nach Anspruch 1 oder 2, **dadurch gekennzeichnet, dass** die Zusammensetzung für eine orale Verabreichung bestimmt ist.

5. Kosmetische nicht-therapeutische Verwendung mindestens eines Öldestillats von Sonnenblumenöl zur Behandlung von Problemen, die mit der Alterung der Haut, der benachbarten Schleimhäute und/oder der Hautanhanggebilde verbunden sind, oder zur Behandlung von Problemen, die mit der Austrocknung der Haut, der benachbarten Schleimhäute und/oder der Hautanhanggebilde verbunden sind.

6. Kosmetische Verwendung nach Anspruch 5, **dadurch gekennzeichnet, dass** das Öldestillat von Sonnenblumenöl für eine topische Anwendung bestimmt ist.

7. Kosmetische Verwendung nach Anspruch 5, **dadurch gekennzeichnet, dass** das Öldestillat von Sonnenblumenöl für eine orale Verabreichung bestimmt ist.

## Claims

1. Use of at least one sunflower oil oleodistillate, for the preparation of a composition for treating dry skin, skin exposed to actinic rays, notably UV rays, sensitive skin, irritated skin, reactive skin, cutaneous desquamation, pruritus, ichthyosis, acne, xerosis, or atopic dermatitis.

2. Use according to claim 1, **characterised in that** the sunflower oil oleodistillates are chosen from the group consisting of oleodistillates rich in tocopherols and/or phytosterols.

3. Use according to claim 1 or 2, **characterised in that** the composition is for topical application.

4. Use according to claim 1 or 2, **characterised in that** the composition is for oral administration.

5. Non-therapeutic cosmetic use of at least one sunflower oil oleodistillate, for treating disorders associated with ageing of the skin, neighbouring mucosa and/or skin appendages or for treating disorders associated with drying of the skin, neighbouring mucosa and/or skin appendages.

6. Cosmetic use according to claim 5, **characterised in that** the sunflower oil oleodistillate is for topical application.

7. Cosmetic use according to claim 5, **characterised in that** the sunflower oil oleodistillate is for oral administration.
